(19) Europäisches Patentamt
European Patent Office
Office européen des brevets

(11) Veröffentlichungsnummer : **0 079 021**
**B1**

# (12) EUROPÄISCHE PATENTSCHRIFT

(45) Veröffentlichungstag der Patentschrift : **05.03.86**

(51) Int. Cl.4 : **C 07 D233/76**, C 08 G 73/10, C 09 D 3/49

(21) Anmeldenummer : **82110067.4**

(22) Anmeldetag : **02.11.82**

## (54) Verfahren zur Herstellung von (Thio)Hydantoinen.

(30) Priorität : **11.11.81 DE 3144700**

(43) Veröffentlichungstag der Anmeldung : **18.05.83 Patentblatt 83/20**

(45) Bekanntmachung des Hinweises auf die Patenterteilung : **05.03.86 Patentblatt 86/10**

(84) Benannte Vertragsstaaten : **AT DE FR GB IT**

(56) Entgegenhaltungen :
**EP-A- 0 002 662**
**EP-A- 0 033 477**
**Chemical Abstracts Band 74, Nr. 17, 26. April 1971, Columbus, Ohio, USA M. ROBBA et al. "Mechanism of cyclization of carbodiimides with alpha-hydroxy carboxylic acids" Seite 369, Spalte 1, Abstract Nr. 87082c**

(73) Patentinhaber : **BAYER AG**
**Konzernverwaltung RP Patentabteilung**
**D-5090 Leverkusen 1 Bayerwerk (DE)**

(72) Erfinder : **Schulte, Bernhard, Dr.**
**Suedwall 80 A**
**D-4150 Krefeld (DE)**
Erfinder : **Jakob, Wolfgang**
**Am Domacker 81**
**D-4130 Moers 1 (DE)**
Erfinder : **Dünwald, Willi, Dr.**
**Geschwister-Scholl-Strasse 16**
**D-5090 Leverkusen 1 (DE)**
Erfinder : **Meyer, Karl-Heinrich, Dr.**
**Deswatinesstrasse 89**
**D-4150 Krefeld (DE)**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Die Erfindung betrifft ein Verfahren zur Herstellung von Verbindungen mit mindestens einem (Thio) Hydantoinring im Molekül durch Umsetzung von Carbodiimiden mit Hydroxybernsteinsäureestern bzw. Mercaptobernsteinsäureestern.

Hydantoine, Polyhydantoine und Verfahren zu ihrer Herstellung sind bekannt (Am. Chem. J. *45*, 383 ; BE-PS 678 282).

Niedermolekulare Hydantoine werden bevorzugt im Pharma- und Pflanzenschutzbereich verwendet, höhermolekulare Hydantoine sind beispielsweise für wärmebeständige Beschichtungsmittel von Bedeutung (FR-PS 1 484 694).

Gegenstand der Erfindung ist ein neues Verfahren zur Herstellung von (Thio) Hydantoinringe enthaltenden Verbindungen, das dadurch gekennzeichnet ist, daß man ein Carbodiimid mit Hydroxy- oder Mercaptobernsteinsäurediestern der Formel

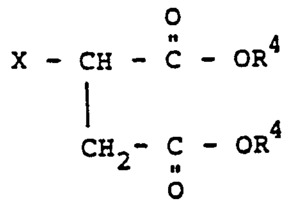

in welcher

$R^4$ für eine Alkyl-, Cycloalkyl- oder Benzylgruppe und

X für eine Hydroxy- oder Mercapto-Gruppe steht,

bei Temperaturen von ca. 20 bis ca. 250 °C, gegebenenfalls in Gegenwart eines Katalysators und gegebenenfalls in Anwesenheit eines Lösungsmittels, umsetzt.

Es ist bekannt; daß sich Carbodiimide bei der Wahl geeigneter Katalysatoren mit Alkoholen zu O,N,N'-trisubstituierten Isoharnstoffen (vgl. z. B. Chem. Rev., *67*, 2 (1967) S. 107 ; Ann. *597*, 235 (1955) ; Chem. Abstr., *54*, 5471 (1960)) und mit Thioalkoholen zu den S-substituierten Isothioharnstoffen (vgl. Ann., *661*, 164 (1963)) umsetzen. Weiterhin ist aus Ann., *639*, 24 (1961) bekannt, daß die Umsetzung von aliphatischen Carbodiimiden mit α-Hydroxycarbonsäure-estern, z. B. mit den Methyl- und Ethylestern der Glykol-, Milch- und α-Hydroxyisobuttersäure zu den 2-Alkylimino-3,5-dialkyl-4-oxazolidonen führt. Hydantoine werden bei diesen Umsetzungen nicht erhalten.

Die (Thio) Hydantoinbildung gelingt, wenn man nach den erfindungsgemäßen Bedingungen Carbodiimide mit speziellen α-Hydroxycarbonsäureestern wie Hydroxybernsteinsäurediestern (Äpfelsäureestern) bzw. Sulfhydrylbernsteinsäurediestern vorzugsweise in Gegenwart von Katalysatoren, die die Addition von OH-funktionellen Verbindungen an die Carbodiimidgruppe beschleunigen, umsetzt.

Besonders vorteilhaft gelingt nach dem erfindungsgemäßen Verfahren die Synthese von Polyhydantoinen in nichtphenolischen Lösungsmitteln, wobei Polymere mit sehr hohen Erweichungstemperaturen erhalten werden. Diese sind als hochtemperaturbeständige Beschichtungsmittel, insbesondere auf dem Sektor der Elektroisolierlacktechnik, geeignet oder können auch speziell zur Erhöhung der Erweichungstemperaturen und Verbesserung der Verlaufseigenschaften anderer bekannter Isolierstoffe dienen.

Als Carbodiimid-Verbindungen im Sinne der Erfindung können Monocarbodiimide mit einer —N=C=N-Gruppe im Molekül, deren cyclische Dimere oder Trimere oder auch lineare bzw. verzweigte Polycarbodiimide mit mehr als zwei Carbodiimid-Gruppen im Molekül verwendet werden.

Bevorzugt sind Carbodiimide der Formel I und II

$$R^1-N=C=N-R^2 \quad (I) \qquad [\overline{Y}-N=C=\underline{N}]_n \quad (II)$$

in denen

$R^1$ und $R^2$ gleich oder verschieden einen aliphatischen Rest mit 1-20 C-Atomen, einen cycloaliphatischen Rest mit 5-12 C-Atomen, einen aliphatisch-aromatischen Rest mit 6-20 C-Atomen, einen aromatischen Rest mit 6-16 C-Atomen, einen Heteroatome wie N, O oder S enthaltenden aromatischen oder cycloaliphatischen Rest mit 5-12 C-Atomen, die jeweils gegebenenfalls mit Halogen (Chlor, Brom, Jod, Fluor), Nitril-, $C_2$-$C_{12}$-Dialkyl-amino-, $C_7$-$C_{12}$-Alkylarylamino-, $C_2$-$C_{18}$-Alkoxycarbonyl-, $C_7$-$C_{18}$-Aroxycarbonyl-, $C_2$-$C_{18}$-Alkylcarboxy-, $C_7$-$C_{18}$-Arylcarboxy-, $C_1$-$C_{18}$-Alkoxy-, $C_6$-$C_{18}$-Aroxy-, $C_1$-$C_{18}$-Alkyl- bzw. Halogenalkyl- oder Nitrogruppen substituiert sein können, oder einen $C_2$-$C_{12}$-Dialkylamino-, $C_2$-$C_{10}$-Alkoxycarbonyl-, $C_6$-$C_{18}$-Glykosylrest oder eine —$Si(R^3)_3$-, —$Sn(R^3)_3$-, —$SO_2R^3$-Gruppe (mit $R^3 = C_6$-$C_{12}$-Aryl bzw. $C_1$-$C_8$-Alkyl) bedeuten oder miteinander als Glieder entsprechend cyclischer organischer Reste verknüpft sein können, und

Y die für $R^1$, $R^2$ angegebene Bedeutung hat und dabei bevorzugt für aliphatische Reste mit 2-12 C-Atomen, cycloaliphatische Reste mit 5-12 C-Atomen, $C_6$-$C_{16}$-Arylreste oder über O, S, $SO_2$, $CH_2$, $CH_3$—C—$CH_3$ oder CO verknüpfte Diphenylreste oder für —$Si(R^3)_2$-, —$Sn(R^3)_2$-Gruppen steht, und

n eine ganze Zahl von 2 bis 2 000, vorzugsweise 2 bis 1 000 bedeutet.

Als Monocarbodiimide werden erfindungsgemäß N,N'-symmetrisch und/oder asymmetrisch substituierte aliphatische aliphatisch-aromatische, cyclische, heterocyclische, aromatische, gegebenenfalls durch Heteroatome substituierte Verbindungen mit einer —N = C = N-Gruppe im Molekül eingesetzt, z. B. Dialkylcarbodiimide wie Dimethyl-, Diethyl-, Diisopropyl-, Dihexyl-, Dibutyl-, Methyl-, tert.-butyl-, Dinonyl-, Didodecyl- und Distearylcarbodiimid, vorzugsweise aromatische, gegebenenfalls substituierte Monocarbodiimide wie Diphenyl-, Ditolyl-, Dinaphthylcarbodiimid, Di-p-iodphenyl-, Di-p-bromphenyl-, Di-p-dimethylaminophenyl-, Di-pyridyl-carbodiimid, Di-Nitro-, -Alkoxy-, -Aroxy-, -Chlor-, -Dichlor-, -Trichlor-, -Tetrachlor-, -Pentachlorphenyl-, -Benzylcarbodiimid oder Carbodiimid-dibenzoesäureester, -diphthalsäureester, -di-isophthaläureester, Carbodiimid-dibenzonitril, cyclo-aliphatische Carbodiimide wie Dicyclohexylcarbodiimid und ungesättigte Carbodiimide wie Diallyl-, Dioleyl-, Dicyclohexenyl-carbodiimid.

Diese Carbodiimid-Verbindungen können nach bekannten Verfahren z. B. aus den entsprechenden Thioharnstoffen in Gegenwart von Metalloxiden, Quecksilbersalzen, Natriumsalzen, Arylsulfochloriden bzw. durch Oxidation von Thioharnstoffen oder aus S-Alkylisothioharnstoffen, Harnstoff-Verbindungen, wie z. B. in Chem. Rev., *67*, 2 (1967), S. 107 angegeben oder aus den entsprechenden Isocyanat-Verbindungten unter Kohlendioxidabspaltung in Gegenwart der bekannten speziellen Katalysatoren zur $CO_2$-Abspaltung hergestellt werden (FR-PS 1 180 307).

Weiterhin können die N-Sulfonylcarbodiimide $RSO_2N = C = NR$, die N-Aminocarbodiimide $RN = C = N—NR_2$ oder die N,N'-Disilylcarbodiimide, wie diese z. B. in Chem. Rev., *67*, 2 (1967), S. 107 ; Angew. Chem., *77*, 430 (1965) ; J. Org. Chem., *29*, 2816 (1964) ; Ann., 652, 21 (1962) ; Z. Anorg. Allgem. Chem., *330*, 101 (1964) aufgeführt sind, eingesetzt werden.

Als erfindungsgemäß einzusetzende Ausgangskomponenten kommen ebenso aliphatische, cycloaliphatische, araliphatische, aromatische und heterocyclische lineare oder verzweigte Polycarbodiimide mit mehr als 2 Carbodiimidgruppen in Betracht, sowie deren Gemische oder Polycarbodiimide, die eine statistische Zusammensetzung oder einen blockartigen Aufbau aus unterschiedlichen Strukturelementen in bestimmter Sequenzlänge im Polymermolekül aufweisen und somit die oben angeführten aliphatischen, cycloaliphatischen, araliphatischen, aromatischen und heterocyclischen Struktureinheiten in unterschiedlichsten Verhältnissen sowohl statistisch verteilt als auch blockweise angeordnet im Polymermolekül enthalten können.

Im Falle der Synthese dieser Polycarbodiimide mit zwei oder mehr Carbodiimidgruppen im Molekül aus mehrfunktionellen Isocyanaten können die aus der Literatur bekannten Katalysatoren (vgl. z. B. FR-PS 1 180 307), beispielsweise Phospholine, Phospholidinsulfide oder auch metallorganische Verbindungen der Gruppe Ia-IIIa, wie z. B. Phenyllithium, Diethylzink eingesetzt werden.

Dabei können die erfindungsgemäßen Polycarbodiimid-Verbindungen aus Polyisocyanaten, wie sie z. B. in Annalen 562, S. 75-136 ; Am. Chem. J. 45, 383 ; DE-OS 29 08 626 ; DE-OS 27 14 655 ; EP-PS 0012379 umfassend aufgeführt sind, hergestellt werden.

Besonders bevorzugt sind Gemische aus Poly-Toluylen-Carbodiimiden (2,4- und 2,6-Substitutionsprodukte) oder aus Poly-p- und -m-phenylencarbodiimiden, sowie Polycarbodiimide auf der Basis von Anilin-Formaldehydkondensaten mit Polyphenylenmethylen-Struktur und Poly-4,4'-diphenylmethan-, Poly-4,4'-diphenylether-, Poly-p-phenylen-, Poly-naphthylencarbodiimid, Polyisophoroncarbodiimid, Polyhexamethylencarbodiimid, Polycumencarbodiimid, Polymesitylencarbodiimid und/oder deren Gemische sowie Blockpolycarbodiimide z. B. der folgenden Strukturen ;

—B—B—B—A—A—A—A—B—B—B—

—C—C—B—B—B—A—A—A—A—B—B—B—C—C—

wobei A beispielsweise ein aromatisches Strukturelement wie Diphenylmethan ist, B einen aliphatischen Rest, wie z. B. den Isophoronrest und C eine aromatische Einheit, wie z. B. die Toluylen- oder Naphthylengruppe darstellt, die über Carbodiimidgruppen miteinander verknüpft sind.

Diese Blockpolycarbodiimide können z. B. hergestellt werden, indem die Carbodiimidisierung der einzelnen verwendeten mehrfunktionellen Isocyanate stufenweise nacheinander erfolgt. Die aufgeführten Strukturen und technisch leicht zugänglichen bisfunktionellen Isocyanate verdeutlichen die Variationsbreite im Hinblick auf Sequenzlängen und Mengenverhältnisse der einzelnen Bausteine, wobei die Polycarbodiimide auch gezielt verzweigt werden können, wenn z. B. tri- und mehrfunktionelle Isocyanate in den Carbodiimidisierungsstufen eingesetzt werden.

Als erfindungsgemäß einzusetzende α-Hydroxy- bzw. α-Sulfhydrylcarbonsäureester-Verbindungen sind Bernsteinsäureester-Derivate der Formel III

(Siehe Schema Seite 4 f.)

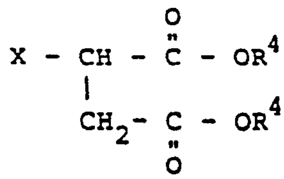

(III)

in der

X für die —OH- bzw. die —SH-Gruppe steht und

$R^4$ eine Alkylgruppe mit 1 bis 18 C-Atomen, vorzugsweise den Methyl-, Ethyl-, Propyl-, Isopropyl-, n-Butylrest, oder einen $C_5$-$C_{12}$-cycloaliphatischen Rest, vorzugsweise die Cyclohexylgruppe oder den Benzylrest bedeutet, geeignet.

Besonders bevorzugt werden die Methyl-, Ethyl-, Cyclohexyl- und Benzyldiester der Hydroxybernsteinsäure eingesetzt. Die im erfindungsgemäßen Verfahren einsetzbaren Ester können beispielsweise durch Veresterung von Äpfelsäure mit den entsprechenden Alkoholen hergestellt werden.

Das erfindungsgemäße Verfahren läßt sich beispielhaft durch folgende Gleichung wiedergeben :

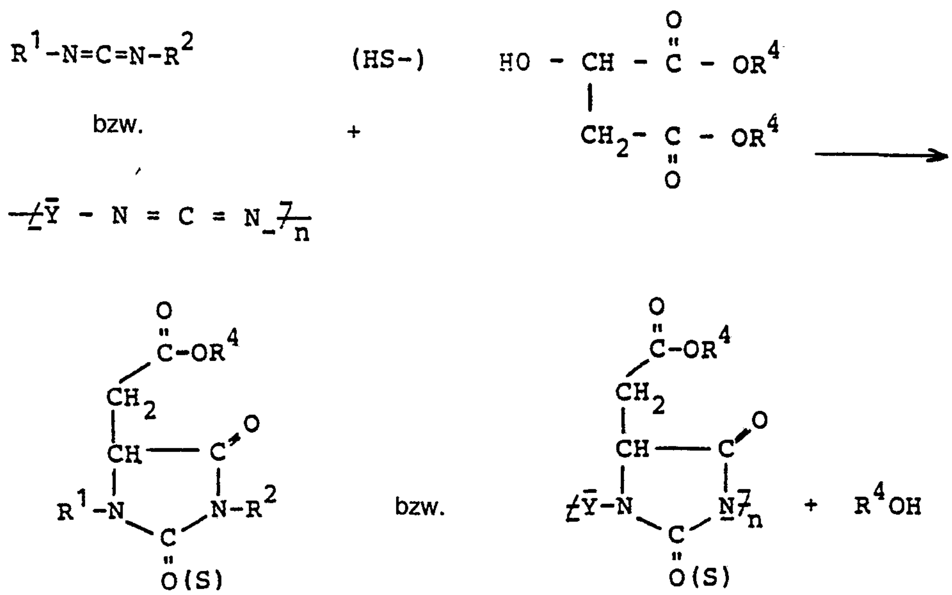


Im allgemeinen werden pro Äquivalent Carbodiimid mindestens 1 Val Hydroxy- bzw. Sulfhydrylbernsteinsäureester eingesetzt. Doch sind auch sehr weitgehende Abweichungen von diesen stöchiometrischen Mengenverhältnissen möglich.

Wird von den stöchiometrischen Mengenverhältnissen abgewichen, können Modifizierungen anderer Beschichtungsmittel durch das erfindungsgemäße Verfahren erzielt werden.

Die erfindungsgemäßen Hydantoine können durch NMR-Spektren oder durch die charakteristischen IR-Absorptionsbanden für Hydantoine bei 1 770, 1 710 und 1 400-1 410 $cm^{-1}$ identifiziert werden. Die polymeren Reaktionsprodukte weisen Lösungsviskositäten von 200 bis 200 000 mPas, vorzugsweise 1 000 bis 100 000 mPas auf, die in 30 gew.-%igen Lösungen beispielsweise mit Phenol, Kresol, γ-Butyrolacton, N-Methylpyrrolidon, Acetophenon, Benzoesäurealkylestern, Benzylalkohol und deren Gemischen bei 25°C gemessen werden.

Die erfindungsgemäße Umsetzung kann in phenolischen und nichtphenolischen Umsetzungsmedien die unter den Umsetzungsbedingungen inert sind oder gegebenenfalls lockere weiterreagierende Additionsverbindungen bilden oder als heterogene Umsetzung in Suspension, d. h. in Gegenwart von inerten Verdünnungsmitteln oder in Substanz oder in einem Überschuß einer der Umsetzungskomponenten durchgeführt werden.

Als nichtphenolische Umsetzungsmedien eignen sich Verdünnungsmittel, beispielsweise inerte organische Flüssigkeiten, wie aliphatische, aromatische Kohlenwasserstoffe, Halogenkohlenwasserstoffe, Heterocyclen, Ester, Lactone, Ketone, Sulfoxide, Sulfone, Ether, substituierte Amide, Nitrile, Phosphorsäureamide. Beispiele dafür sind Cyclohexan, Ligroine, Tetrachlorkohlenstoff, Chloroform, Methylenchlorid, Ethylenchlorid, Tetrachlorethan, Methylethylketon, Diisopropylether, Ethylenglykol-, Diethylenglykoldimethyl- bzw. -diethylether, Dioxan, Tetrahydrofuran, Toluol, Xylole, Chlorbenzol, Dichlorbenzol, Acetophenon, Cyclohexanon, Propylencarbonat, Caprolactam, Caprolacton, Butyrolacton, Glykolmonomethyletheracetat, Dimethylsulfoxid, Tetramethylsulfon, Benzoesäurealkylester, N-Methylpyrrolidon, Dimethylformamid, Dimethylacetamid, Hexamethylphosphorsäuretriamid, Benzonitril und deren Gemische.

Neben diesen Umsetzungsmedien können auch phenolische Umsetzungsmedien wie Phenole, Kresole, Xylenole in Betracht kommen, wobei besonders deren Mischungen mit den oben aufgeführten Umsetzungsmedien geeignet sind. Dabei werden die phenolischen Lösungsmittel so eingesetzt, daß eine Umsetzung mit dem Carbodiimid vermieden wird, d. h. bei der durch Kupfersalze katalysierten Umsetzung mit Hydroxy- bzw. Sulfhydrylbernsteinsäureestern erfolgt ihre Zugabe vorteilhafterweise nach Umsetzung des Carbodiimids.

Bevorzugt werden für die Herstellung von Monohydantoinen Kombinationen aus niedrig- und höhersiedenden Umsetzungsmedien, z. B. Gemische aus Methylenchlorid/Chlorbenzol bzw. o-Dichlorbenzol oder Methylenchlorid/Xylol bzw. Toluol bzw. Phenol oder γ-Butyrolacton verwendet.

Für die Synthese der polymeren Umsetzungsprodukte werden insbesondere ternäre Gemische aus den aufgeführten Lösungs- bzw. Verdünnungsmitteln eingesetzt,wie z. B. Methylenchlorid/Toluol bzw. Xylol/γ-Butyrolacton bzw. N-Methylpyrrolidon oder Phenol bzw. Kresol. Dabei ist die am leichtesten flüchtige Komponente zur Wärmeabfuhr durch Abdestillieren oder als gutes Lösungsmittel für die Polycarbodiimide geeignet. Die höhersiedende Verbindung kann nach Abschluß der Reaktion zum Polyhydantoin noch teilweise oder vollständig in dem Umsetzungsgemisch verbleiben, wie z. B. Toluol, Xylol, Solvesso. Diese Komponenten sind bevorzugt in der Elektroisolierlacktechnik gebräuchliche Verschnittmittel. Die hochsiedenden Komponenten wie Butyrolacton, N-Methylpyrrolidon, Dimethylformamid, Kresol, Phenol oder deren Mischungen stellen die eigentlichen Lösungsmittel für das Umsetzungsprodukt dar.

Als Verschnitt- bzw. Verdünnungsmittel können außerdem Cyclohexan, Solventnaphtha oder nach erfolgter Umsetzung des Carbodiimids mit den Hydroxy- oder Sulfhydrylbernsteinsäureestern auch Hydroxyalkylether oder aliphatische, aliphatisch-aromatische Alkohole, wie z. B. Butanol, Aminoalkohol, Benzylalkohol, Phenoxyethanol, die Methyl-, Ethyl-, Isopropyl-, Butylmonoether des Ethylen-, Diethylen- bzw. Propylenglykols zugesetzt werden, die dann die Herstellung von nicht-phenolischen Polymerlösungen ermöglichen.

Werden Carbodiimide unter katalytischen Bedingungen mit den erfindungsgemäßen Bernsteinsäurediester-Derivaten umgesetzt, erfolgt die Zugabe der alkoholischen Verdünnungsmittel, wenn kein Carbodiimid mehr IR-spektroskopisch festzustellen ist.

Die erfindungsgemäße Umsetzung der Carbodiimide mit den Hydroxy- bzw. Mercaptobernsteinsäurediestern wird bei der Ausführung in nichtphenolischen inerten Umsetzungsmedien, vorzugsweise in Gegenwart von Katalysatoren, die die Addition von OH-funktionellen Verbindungen an die Carbodiimidgruppe beschleunigen, durchgeführt. Besonders bevorzugt werden Kupfer-I- und Kupfer-II-chlorid eingesetzt.

Die weitere Umsetzung zum Hydantoin kann unter Zusatz der für Umlagerungsreaktionen geeigneten Katalysatoren, wie z. B. Iod usw., Saüren wie p-Toluolsulfonsäure, Essigsäure usw. und der für Cyclisierungsreaktionen bekannterweise geeigneten Katalysatoren wie tert.- Amine, z. B. Triethylamin, N-Methylmorpholin, Endoethylenpiperazin, Metallverbindungen wie Titantetrabutylat, Titanaminoalkohol, Eisenacetylacetonat, Dibutylzinnlaurat, Bleiacetat, Bleinaphthenat, Bleiethylhexoat, Zinnoctoat oder Calciumnaphthenat durchgeführt werden. Als Katalysatoren kommen auch stickstoffhaltige Basen, wie Tetraalkylammoniumhydroxide, ferner Alkalihydroxide wie Natriumhydroxid, Alkaliphenolate wie Natriumphenolat oder Alkalialkoholate wie Natriummethylat in Betracht.

Wird die erfindungsgemäße Umsetzung von Carbodiimiden mit den Hydroxy- bzw. Mercaptobernsteinsäurediestern in phenolischen Umsetzungsmedien, beispielsweise in Kresol durchgeführt, können ohne Einsatz von Katalysatoren, beispielsweise ohne Verwendung von Kupfer-Salz Katalysatoren, die Hydantoine direkt erhalten werden.

Werden Carbodiimide eingesetzt, die aus Isocyanaten in bekannter Weise (vgl. z. B. DE-AS-1 122 057 ; US-PS-2 941 983) hergestellt worden sind, kann direkt dieses Umsetzungsgemisch einschließlich des darin enthaltenen Katalysators, benutzt werden.

Bei der Durchführung des erfindungsgemäßen Verfahrens werden die Carbodiimide, vorzugsweise gelöst oder suspendiert in einem Umsetzungsmedium, zusammen mit den Hydroxy- bzw. Sulfhydrylbernsteinsäurediestern vorzugsweise in Gegenwart von Kupfer-I oder Kupfer-II-chlorid einige Minuten bis zu mehreren Stunden bei Temperaturen von ca. 20 bis ca. 250 °C, bevorzugt von 30 bis 200 °C gehalten.

Der Verlauf der Umsetzung kann beispielsweise IR-spektroskopisch verfolgt werden.

Die Umsetzungskomponenten werden bevorzugt bei Temperaturen von 20 bis 150 °C, besonders bevorzugt bei 30 bis 100 °C, gegebenenfalls unter Außenkühlung, in der Weise zusammengebracht, daß eine der Umsetzungskomponenten zur vorgelegten anderen Umsetzungskomponente gegeben wird.

Bei dieser Ausführung können sowohl die Carbodiimide als Lösungen oder Suspensionen als auch die Hydroxy- bzw. Sulfhydrylbernsteinsäureester mit oder ohne weitere Lösungsmittel vorgelegt werden.

Vorteilhafterweise werden bei einer technischen Polyhydantoinherstellung im Falle der Carbodiimidisierung von mehrfunktionellen Isocyanaten die Carbodiimide vorgelegt, da sie nach Abschluß ihrer Herstellung sofort weiter zum erfindungsgemäßen Hydantoin umgesetzt werden können.

Werden Polycarbodiimide eingesetzt, die einen blockartigen Aufbau —B—A—B— aufweisen, wobei A und B Sequenzen von jeweils verschiedenen Kettengliedern darstellen, wird im allgemeinen so verfahren, daß ein Diisocyanat (z. B. 4,4'-Diisocyanatodiphenylmethan) in den aufgeführten, für die Herstellung von Monohydantoinen geeigneten Reaktionsmedien aus einer leicht- und schwerer flüchtigen

Flüssigkeit, wie z. B. Methylenchlorid/Toluol bis zum nahezu vollständigen Umsatz carbodiimidisiert wird. Anschließen wird, gegebenenfalls unter Zusatz von weiterem Lösungs-/Verdünnungsmittel, das Diisocyanat- oder auch z. B. Triisocyanat — mit der Strucktureinheit B (z. B. Naphthyl-, Isophoron-, Tolyl-) — zugegeben und die Carbodiimidisierung fortgesetzt, wobei dann mit Vorteil Monoisocyanate (z. B. Phenyl-, Tolyl-, Naphthyl-, Cyclohexyl-, Methyl-, Cyclohexenyl-, Oleylisocyanat, Isocyanatobenzoesäureester, -phthalsäureester, -isophthalsäureester usw.) im Verlauf des weiteren Polycarbodiimidaufbaues als Regler eingetragen werden oder auch OH- bzw. NH-funktionelle Verbindungen zur Regelung des Molekulargewichts der Polycarbodiimide verwendet werden können.

Anschließend können die Hydroxy- bzw. Sulfhydrylbernsteinsäurediester, gegebenenfalls gelöst in den angegebenen geeigneten Umsetzungsmedien, zugesetzt werden. Dabei wird die Umsetzung zum Hydantoin mit Vorteil durch stufenweise Steigerung der Reaktionstemperatur erreicht.

Die Umsetzung wird zweckmäßigerweise unter einem inerten Schutzgas wie $N_2$ oder Argon durchgeführt.

Die erfindungsgemäße Reaktion kann kontinuierlich oder diskontinuierlich bei Normaldruck oder bei Überdruck ausgeführt werden.

Die Aufarbeitung der niedermolekularen Reaktionsprodukte kann nach gängigen Methoden wie z. B. Kristallisation oder Umfällen erfolgen.

Die nach dem erfindungsgemäßen Verfahren zugänglichen monomolekularen Hydantoine zeigen Wirkungen auf dem pharmazeutischen und Pflanzenschutzsektor.

Die erfindunsgemäßen Polyhydantoine zeichnen sich durch besonders hohe Temperaturbeständigkeiten aus. Es werden z. B. bei der Prüfung nach DIN 46 453 eines mit diesen Polyhydantoinen beschichteten Kupferdrahtes Erweichungstemperaturen von über 400 °C festgestellt. Weiterhin zeigen die polymeren Reaktionsprodukte eine gute Löslichkeit und ausgezeichnete Verlaufseigenschaften bei der Beschichtung elektrischer Leiter und sind geeignet zur Verwendung als Kleber, Lacke, Folien, Pulver, Fasern und Formkörper, wobei ihre Eigenschaften durch Zusatz von Füllstoffen, Pigmenten und nieder- und/oder hochmolekularen Komponenten z. B. zur Herstellung von Lacken und Folien in weiten Grenzen variiert werden können.

Die nach dem erfindungsgemäßen Verfahren modifizierten bekannten Polykondensate, wie z. B. Polyurethane, Polyamidimide, Polyester, Polyesterimide, Polyesteramidimide, Polyhydantoine, Polycarbonate, Polyamide usw., die zugemischt oder gegebenenfalls unter Mitverwendung von z. B. Polycarbonsäuren, deren Anhydriden, Estern, Polyolen usw. bei der erfindungsgemäßen Herstellung der beanspruchten Polyhydantoine an- und/oder einkondensiert werden, wie diese Modifizierungsreaktionen z. B. aus DE-OS-2 908 626 oder DE-OS-2 714 655 bekannt sind, zeigen verbessertes Temperaturverhalten und wesentlich verbesserte Verlaufseigenschaften, wenn derart modifizierte Produkte zur Beschichtung hitzeresistenter Substrate eingesetzt werden.

Insbesondere geeignet sind die erfindungsgemäßen Hydantoine im Falle ihrer Herstellung in kresolfreien Lösungsmitteln zur Modifizierung kresolfrei löslicher Trishydroxyethylenisocyanurat enthaltender Polyester, wobei die bekannterweise kritischen Verlaufseigenschaften dieser Polyester, aber auch deren Erweichungstemperaturen wesentlich verbessert werden und nichtphenolische Lacklösungen mit ausgezeichnetem Werteniveau bei der Drahtlackierung erhalten werden.

Die Mengenverhältnisse dieser Zusatzstoffe können je nach beabsichtigem Verwendungszweck sehr unterschiedlich sein, vorzugsweise werden bezogen auf die erfindungsgemäßen Polyhydantoine 5 bis 500 Gew.-% eingesetzt.

Bevorzugt verwendet werden die erfindungsgemäßen Polyhydantoine für Einbrennlacke, insbesondere Draht- bzw. Elektroisolierlacke, wobei der Festgehalt der möglichen Lacklösungen bzw. Lackmischungen in weiten Grenzen schwanken kann und sowohl vom Lösungsmittelverhalten der Bindemittel als auch vom beabsichtigten Einsatzzweck bestimmt wird. Vorzugsweise liegt der Feststoffgehalt im Bereich von 20 bis 75 Gew.-%. Die Polyhydantoine können als Lösungen auf den üblichen Lackiermaschinen sowie als Tränklacke bis 60 % Feststoffgehalt, gegebenenfalls auch aus der Schmelze oder als Pulver, verarbeitet werden.

## Beispiele

### Beispiel 1

49,7 g einer 19,5 gew.-%igen Diphenylcarbodiimidlösung (0,05 Mol) in γ-Butyrolacton mit den typischen IR-Absorptionsbanden bei 2 110/2 140 $cm^{-1}$ werden vorgelegt.

Bei 60 °C werden innerhalb von 10 Min. 8,1 g (0,05 Mol) Äpfelsäuredimethylester (Hydroxybernsteinsäuredimethylester) eingetropft. Innerhalb von 2 Std. wird auf 100 °C aufgeheitzt. Das IR-Spektrum zeigt, daß das Diphenylcarbodiimid noch nicht umgesetzt ist. Nach dem Abkühlen auf Raumtemperatur werden 30 mg Kupfer (I)-chlorid zugesetzt, und nach 15 Min. bei 60 °C wird kein Carbodiimid im IR-Spektrum mehr festgestellt. Innerhalb einer Stunde wird auf 100 °C aufgeheizt und nach Zugabe von 20 mg Diaza-bicyclooctan und 10 g Toluol wird die Reaktionstemperatur innerhalb von 4 Stunden auf 160 °C erhöht, wobei die Übergangstemperatur auf 68 °C ansteigt. Es wird 2 Stunden bei 180 °C nachgerührt. Die gaschromatographische Analyse des Destillats ergibt eine Methanolmenge von 1,2 g (auf der Basis der Flächenprozente). Nach Filtration der Reaktionslösung wird aus 300 ml $H_2O$ ausgefällt, wobei nach

der Vakuumtrocknung 11,6 g eines bräunlichen Produkts erhalten werden, daß im IR-Spektrum die typischen Hydantoin- und Esterbanden aufweist (1 770 $cm^{-1}$, Doppelbande bei 1 730/1 715 $cm^{-1}$ und Ringbande bei 1 410 $cm^{-1}$). Nach der Umkristallisation aus Methanol werden in NMR-Spektrum ($CDCl_3$) folgende Protonen-Signale festgestellt :

| | | | |
|---|---|---|---|
| 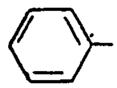 | Chem. Verschiebung | $\delta$ = | 7,1-7,6 ppm |
| $-\dot{C}H$ | " " | $\delta$ = | 4,8-4,95 ppm (Triplett) |
| $-OCH_3$ | " " | $\delta$ = | 3,6-3,65 ppm (Singlett) |
| $-CH_2-CO-$ | " " | $\delta$ = | 2,9-3,0 ppm (Triplett) |

Die Integralkurven des NMR zeigen in der angegebenen Reihenfolge der Protonen ein Verhältnis von 10 : 1 : 3 : 2, womit die Struktur des 1,3-Diphenyl-hydantoyl-5-essigsäuremethylesters bestätigt wird.

## Beispiel 2

41,2 g Polydiphenylmethancarbodiimid (0,2 Mol) mit 0,025 Mol Phenylcarbodiimid-Endgruppen in 150 g Chlorbenzol (hergestellt durch Carbodiimidisierung von 47 g 4,4'-Diisocyanatodiphenylmethan unter Zugabe von 0,5 g eines Gemisches aus 1-Methyl-1-phospha-2-cyclopenten-1-oxid und 1-Methyl-1-phospha-3-cyclopenten-1-oxid und 3 g (0,025 Mol) Phenylisocyanat zur Molekulargewichtsbegrenzung in Chlorbenzol unter Erhitzen) werden bei 70-75 °C vorgelegt. Die viskose eingetrübte Polycarbodiimidsuspension weist die charakteristischen IR-Banden bei 2 110/2 140 $cm^{-1}$ auf.

Es werden 50 mg Kupfer-I-chlorid zugesetzt und bei 70-75 °C wird eine Lösung von 32,4 g (0,2 Mol) Hydroxybernsteinsäuredimethylester in 10 g Chlorbenzol und 40 g γ-Butyrolacton homogen eingerührt.

In 30 Min. wird die Reaktionstemperatur durch Nachheizen auf 100-105 °C erhöht. Das Polycarbodiimid ist vollständig umgesetzt. Beim Aufheizen auf 130 °C innerhalb von 1 Std. werden bis zu einer Übergangstemperatur von ca. 100 °C 5,4 g Destillat erhalten, die der gaschromatographischen Analyse entsprechend zu 80 % aus Methanol bestehen. Innerhalb von 2 Stunden wird unter weiterem Abdestillieren und Zugabe von 150 g m-Kresol 70 auf 180 °C aufgeheizt. Nach weiteren 4 Stunden bei 185 °C wird eine Polymerlösung mit einem Festgehalt von 26,8 % (5 Min. bei 360 °C eingebrannt) und einer Viskosität von 270 mPas (15 %ig in m-Kresol bei 20 °C mit einem Höppler-Viskosimeter bestimmt) erhalten. Das aus Methanol ausgefällte Polymere zeigt die für Hydantoinstrukturen typischen Banden bei 1 775, 1 720 und 1 410 $cm^{-1}$.

Beim Lackierversuch eines Kupferdrahtes von 0,7 mm Durchmesser mit dieser Polyhydantoinlösung auf einer Vertikal-Lackiermaschine (Ofenlänge 4 m) werden die für Polyhydantoine bekannten guten Verlaufseigenschaften und nach DIN 46 453 bei Fahrgeschwindigkeiten von 7-10 m/Min. und Einbrenntemperaturen von 400 °C Erweichungstemperaturen von oberhalb 400 °C festgestellt. Bei 7 m/Min. Vorschub werden z. B. Erweichungstemperaturen von über 468 °C, Hitzeschockwerte von 220-260 °C, Außenfaserdehnung bis zu 67 %, Abriebfestigkeiten (NEMA) von 30 und ein elektrisches Durchschlagverhalten von ca. 6 kV für den Lackdraht erzielt.

## Beispiel 3

16,2 g (0,1 Mol) Hydroxybernsteinsäuredimethylester werden als 40 %ige Lösung in 53,4 g m-Kresol 70 bei Raumtemperatur vorgelegt. Nach Zugabe von 45,5 g einer 42,7 gew.-%igen Diphenylmethancarbodiimidlösung in $CH_2Cl_2$ (0,1 Mol) wird 4 Stunden bei 30-35 °C gerührt. Es wird im IR keine Umsetzung des Carbodiimids festgestellt. Man rührt 2 Stunden bei 60 °C, wobei die Reaktionslösung eintrükbt. Nach einer weiteren 1/2 Stunde bei 80 °C wird IR-spektroskopisch immer noch unumgesetztes Diphenylcarbodiimid beobachtet. Unter Abdestillieren des Methylenchlorids wird in ca. 1/2 Stunde auf 110 °C aufgeheizt, die 4 Stunden gehalten werden, wobei nach 3 Stunden bei 110 °C im IR-Spektrum die Carbodiimidbanden bei 2 110/2 140 $cm^{-1}$ nicht mehr, allerdings eine scharfe Bande bei 1 710 $cm^{-1}$ und eine Schulter bei 1 770 $cm^{-1}$ — kennzeichnend für die einsetzende Hydantoinringbildung — festgestellt werden.

Es wird auf 170-175 °C aufgeheizt, die 4 Stunden gehalten werden. Das erhaltene Destillat enthält

84,5 % der theoretischen Methanolmenge. Das Reaktionsprodukt wird als grauweißes Pulver nach dem Ausfällen aus einem Methanol/Wasser-Gemisch erhalten (17,5 g). Nach dem Umkristallisieren aus Methanol stimmen die NMR-Signale mit den unter Beispiel 1 aufgeführten Werten überein. Das unkristallisierte Produkt zeigt IR-Banden bei 1 770/1 730/1 710 und 1 410 $cm^{-1}$ und besitzt einen Schmelzpunkt von 144-147 °C.

Beispiel 4

In 40 g Methylenchlorid und 40 g Toluol sind 22,6 g Polydiphenylmethancarbodiimid mit ca. 0,1 Mol Carbodiimidgruppen und 0,01 Mol Phenylcarbodiimid-Endgruppen — hergestellt durch Carbodiimidisierung von 25 g (0,1 Mol) 4,4'-Diisocyanatodiphenylmethan unter Zugabe von 0,3 g Methylphospholinoxid und 1,2 g Phenylisocyanat als Regler durch Aufheizen auf ca. 45-50 °C — vorgelegt.

Es werden 30 mg Kupfer-I-chlorid und 30 g N-Methylpyrrolidon eingetragen. Man steigert die Sumpftemperatur unter Destillation auf 70 °C, wobei die Rührfähigkeit des Polymeren beobachtet und weiteres N-Methylpyrrolidon zur Verdünnung bereitgehalten wird. Die Lösung zeigt die für die Carbodiimid-Gruppen typischen IR-Banden bei 2 100/2 150 und die schwächere Bande bei 2 040 $cm^{-1}$. Nach Zugabe von weiteren 20 g N-Methylpyrrolidon wird eine Lösung von 16,2 g (0,1 Mol) Hydroxybernsteinsäuredimethylester in 30 g N-Methylpyrrolidon innerhalb von 10 Min. bei ca. 75 °C zugesetzt und in ca. 1 Std. auf 120 °C aufgeheizt, wobei eine klare braunrote Lösung erhalten wird. Innerhalb von 1 1/2 Std. wird die Reaktionstemperatur auf 180 °C erhöht, wobei bei ca. 140 °C eine Probe der Lacklösung die IR-Banden bei 1.770/1 735/1 720 $cm^{-1}$ als Schultern aufweist. Bei 180-190 °C rührt man 3 Std., und die erhaltene Polymerlösung weist im IR die Hydantoinbanden in ausgeprägter Form bei 1 720/1 775 und 1 400 $cm^{-1}$ auf. Die theoretisch zu erwartende Methanolmenge wird nahezu quantitativ im Destillat mit Hilfe eines Gaschromatogramms auf Flächenprozentbasis bestätigt.

Diese nichtphenolische Polyhydantoinlösung besitzt einen Festgehalt von 32,1 % (5 Min. bei 360 °C eingebrannt), wobei dieses Einbrennen auf Metallblechen zu elastischen, klaren rotbraunen Polymerfilmen führt. Die Viskosität der erhaltenen Polyhydantoinlösung beträgt 15 %ig, mit N-Methylpyrrolidon verdünnt, bei 20 °C 440 mPas.

Beispiel 5

A. Als hochviskose Suspension wird ein Polycarbodiimid mit blockartiger Anordnung der Strukturelemente in der Art —B—B—B—A—A—B—B—B— aus 25 Mol % Poly-2,4-(80 %)- und -2,6(20 %)-toluylencarbodiimid (A) und 75 Mol % Polydiphenylmethancarbodiimid (B) mit ca. 0,4 Mol Carbodiimidgruppen — hergestellt durch stufenweise Carbodiimidisierung von 17,4 g (0,1 Mol) des Diisocyanats von A und 75 g (0,3 Mol) des Diisocyanats von B unter Zusatz von 0,75 g Methylphospholinoxid und 3,6 g (ca. 0,03 Mol) Phenylisocyanat als Regler durch Aufheizen in 140 g Methylenchlorid und 140 g Toluol — bei ca. 50 °C vorgelegt.

Es werden 100 mg Kupfer-I-chlorid, 100 g γ-Butyrolacton und 100 g N-Methylpyrrolidon eingetragen. Unter Rühren wird auf 65-70 °C aufgeheizt. Innerhalb von 15 Min. werden bei 60-70 °C 76 g (0,4 Mol) Hydroxybernsteinsäurediethylester in 60 g N-Methylpyrrolidon und 40 g γ-Butyrolacton eingerührt. Man steigert die Reaktionstemperatur unter weiterem Abdestillieren der leichtersiedenden Lösungsmittel-Komponenten innerhalb von 3 Std. auf 175-180 °C, die 4 Std. gehalten werden, wobei im Verlauf des Viskositätsanstiegs mit 20 g Butyrolacton und 40 g Benzylalkohol verdünnt wird.

Man erhält eine nichtphenolische Polymerlösung mit einem Feststoffgehalt von 27 % (5 Min. bei 360 °C eingebrannt) und einer Viskosität von 825 mPas — 15 %ig mit γ-Butyrolacton/Benzylalkohol (1 : 1) verdünnt, bei 20 °C gemessen. Das aus Methanol aus- und umgefällte Polymere zeigt die Hydantoine kennzeichnenden ausgeprägten IR-Absorptionsbanden bei 1 775/1 720/1 410 $cm^{-1}$. Im Drahtlackierversuch (Ofenlänge 4 m, Kupferdraht 0,7 mm Ø, 7-9 m/Min. Vorschub bei 400 °C Einbrenntemperaturen) zeigt diese mit Benzylalkohol auf einem Feststoffgehalt von 24 % verdünnte Polyhydantoinlösung gute Verlaufseigenschaften und nach DIN 46 453 werden Erweichungstemperaturen von oberhalb 400 °C bei sehr guten Abriebfestigkeiten und elektrischen Durchschlagswerten bis zu 7 KV festgestellt.

B. 760 g eines handelsüblichen nicht kresolfreien löslichen Terephthalsäure/Glycerin/Ethylenglykol-Polyesters (4 Mol Terephthalsäure ; Festgehalt ca. 82 % ; OH-Zahl 145 ; Säurezahl 4,8) werden unter Stickstoff in Gegenwart von 0,5 g Butyltitanat und 0,5 g Bleiacetat nach Zugabe von 360 g (1,38 Mol) Trishydroxyethylenisocyanurat bei ca. 160 °C durch insgesamt 12 stündiges Rühren bei 160-200 °C umgesetzt. Nach kurzzeitigem Vakuum-Anlegen gegen Ende der Reaktion wird ein bei Raumtemperatur sprödes Harz mit einem Festgehalt von 88 % (3 Std. bei 200 °C), einer OH-Zahl von 286 und einer Säurezahl von 5-6 erhalten.

C. 204,5 g dieses Theic-Polyesters werden 50 %ig in Carbitol/Xylol (6 : 4) gelöst und mit 250 g der kresolfreien 24 %igen unter A. hergestellten Polyhydantoinlösung vermischt.

Die Applizierung dieser Lackmischung auf einen Kupferdraht (Ø 0,7 mm) mit Hilfe einer Drahtlackiermaschine (Ofenlänge 4 m) zeigt bei Einbrenntemperaturen von 400 °C und 7-10 m/min. Vorschub

gegenüber den Eigenschaften des reinen Theic-Polyesters ein wesentlich besseres Verlaufsverhalten, Erhöhung der Hitzeschockwerte bis 180 °C, Steigerung der Erweichungstemperaturen auf über 330 °C und Verbesserung der Abriebfestigkeiten bei Außenfaserdehnungen bis 88 %, elektrischen Durchschlagswerten bis 8 KV und Alterungswerten von bis zu 7 Tagen bei 180 °C. Die Prüfung erfolgt dabei nach DIN 46 453.

Beispiel 6

Ein Blockpolycarbodiimid mit folgender Gruppierung der Polymerbausteine $(—B—A—A—A—A—)_2$ aus 80 Mol % Polydiphenylmethancarbodiimid (A) und 20 Mol % Polynaphthylencarbodiimid (B) mit ca. 0,3 Mol Carbodiimidgruppen — hergestellt durch stufenweise Carbodiimidisierung von 62,5 g (0,25 Mol) des Diisocyanats von A und 15,3 g (0,065 Mol) des Diisocyanats von B unter Aufheizen in 50 g Methylenchlorid, 80 g Chlorbenzol und 50 g N-Methylpyrrolidon in Gegenwart von 0,5 g Methylphospholinoxid unter Verwendung von 3,8 g (0,035 Mol) m-Kresol 70/50 mg Endoethylenpiperazin zur Molekulargewichtsbegrenzung — wird bei 50-55 °C vorgelegt.

Es werden 100 mg Kupfer-I-chlorid zugesetzt und innerhalb von 30 Min. werden, beginnend bei 60 °C, 57 g (0,3 Mol) Hydroxybernsteinsäureethylester in 50 g N-Methylpyrrolidon und 80 g γ-Butyrolacton unter Aufheizen bis auf 100-105 °C eingetragen. Es ist die Rührfähigkeit der hochviskosen Reaktionslösung zu beachten und weitere 50 g N-Methylpyrrolidon werden zur Verdünnung im Verlauf der Temperatursteigerung zugesetzt. Nach ca. 15 Min. bei 100-105 °C wird im IR kein Carbodiimid mehr festgestellt. Man trägt 40 g m-Kresol 70 ein und erhitzt unter weiterem Abdestillieren der leichterflüchtigen Lösungsmittel innerhalb von 2 1/2 Std. auf 180 °C, wobei weitere 30 g m-Kresol 70 zudosiert werden. Bei ca. 180 °C wird 4 Std. gerührt. Im erhaltenen Destillat wird gaschromatographisch fast quantitativ die theoretisch zu erwartende Ethanolmenge festgestellt.

Die Polymerlösung weist einen Feststoffgehalt von 28,5 % (5 Min. bei 360 °C) und eine Viskositsät von 630 mPas (15 %ig mit Kresol verdünnt, bei 20 °C gemessen) auf. Das aus Methanol ausgefällte Polymere zeigt die für Hydantoine typischen IR-Absorptionsbanden (1 775/1 720/1 400 $cm^{-1}$). Das Einbrennen von Proben dieser Lacklösung bei 360 °C auf Metallblechen führt zu klaren elastischen Lackfilmen.

**Patentansprüche**

1. Verfahren zur Herstellung von mindestens einem (Thio) Hydantoinring enthaltenden Verbindungen, dadurch gekennzeichnet, daß man ein Carbodiimid mit Hydroxy- bzw. Mercaptobernsteinsäurediestern der Formel

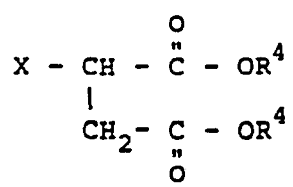

in welcher

$R^4$ für eine Alkyl-, Cycloalkyl- oder Benzylgruppe und

X für eine Hydroxy- oder Mercaptogruppe steht,

bei Temperaturen von ca. 20 bis ca. 250 °C, gegebenenfalls in einem Lösungsmittel und gegebenenfalls in Anwesenheit eines Katalysators, umsetzt.

2. Verfahren nach Anspruch 1, dadurch gekennzeichnet, daß man zur Herstellung von Polyhydantoinen als Carbodiimid ein lineares oder verzweigtes Polycarbodiimid einsetzt.

3. Verwendung der nach Anspruch 2 erhaltenen Polyhydantoine als hochtemperaturbeständige Beschichtungsmittel, Folien, Pulver, Kleber, Lacke oder Formkörper.

**Claims**

1. A process for the production of compounds containing at least one (thio) hydantoin ring, characterised in that a carbodiimide is reacted with hydroxy- or mercapto-succinic acid diesters of the formula :

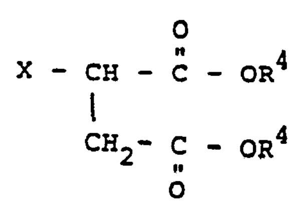

wherein

$R^4$ represents an alkyl, cycloalkyl or benzyl group, and

X represents a hydroxy or mercapto group,

at temperatures of from about 20 to about 250 °C, optionally in a solvent and optionally in the presence of a catalyst.

2. A process according to claim 1, characterised in that a straight or branched polycarbodiimide is used as carbodiimide for the production of polyhydantoins.

3. The use of the polyhydantoins obtained according to claim 2 as high temperature-resistant coating agents, films, powders, adhesives, lacquers or mouldings.

**Revendications**

1. Procédé de production de composés contenant au moins un noyau de (thio) hydantoïne, caractérisé en ce qu'on fait réagir un carbodiimide avec des diesters d'acide hydroxy- ou mercapto-succinique de formule

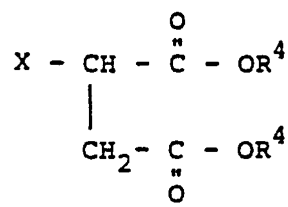

dans laquelle

$R^4$ est un groupe alkyle, cycloalkyle ou benzyle et

X est un groupe hydroxy ou mercapto,

à des températures d'environ 20 à environ 250 °C, éventuellement dans un solvant et le cas échéant en présence d'un catalyseur.

2. Procédé suivant la revendication 1, caractérisé en ce qu'on utilise comme carbodiimide un polycarbodiimide linéaire ou ramifié pour l'obtention de polyhydantoïnes.

3. Utilisation des polyhydantoïnes obtenues suivant la revendication 2 comme agents d'enduction, feuilles, poudres, adhésifs, vernis ou pièces moulées stables aux hautes températures.